# EUROPEAN PATENT APPLICATION

(11) **EP 3 449 978 A1**
(43) Date of publication of application: **06.03.2019**
(21) Application number: 17020402.8
(22) Date of filing: 01.09.2017
(51) Int. Cl.: A61P 35/00, A61K 31/035, A61K 31/352, A61K 31/415, A61K 31/52, A61K 31/7088, A61K 31/713, G01N 33/00

(54) **INHIBITORS OF ARYL HYDROCARBON RECEPTOR FOR TREATING SOFT-TISSUE SARCOMA AND PREVENTING NEUROFIBROMA GROWTH AND/OR TRANSFORMATION TO MALIGNANT PERIPHERAL NERVE SHEATH TUMORS**

(71) Applicant: UNIVERSITE PARIS DESCARTES, 75270 Paris Cedex 06 (FR); Institut National de la Santé et de la Recherche Médicale, 75013 Paris (FR); ASSISTANCE PUBLIQUE, HOPITAUX DE PARIS, 75004 Paris (FR)
(72) Inventor: MASSAAD, Charbel, 94110 Arcueil (FR); PASMANT, Eric, 75013 Paris (FR); SAMPATHKUMAR, Nirmal Kumar, 75014 Paris (FR); SHACKLEFORD, Ghjuvan'Ghjacumu, 20100 Sartene (FR)
(74) Representative: A.P.I. Conseil

(57) **Abstract**

The invention relates to a direct or indirect inhibitor of aryl hydrocarbon receptor (AhR) pathway for use in preventing neurofibroma growth and/or transformation to Malignant Peripheral Nerve Sheath Tumors (MPNST). The invention also relates to a direct or indirect inhibitor of aryl hydrocarbon receptor (AhR) pathway for use in treating soft-tissue sarcoma. In other aspect, the invention relates to the use of aryl hydrocarbon receptor (AhR) pathway in screening methods for identifying new candidate substance or test compound for neurofibroma growth and/or transformation to MPNST, or soft-tissue sarcoma treatment.

## Description

### FIELD OF THE INVENTION

The invention relates to the field of medicine and more particularly to the field of cancer. More specifically, the invention relates to a direct or indirect inhibitor of aryl hydrocarbon receptor (AhR) pathway for use in preventing neurofibroma growth and/or transformation to Malignant Peripheral Nerve Sheath Tumors (MPNST). The invention also relates to a direct or indirect inhibitor of AhR pathway for use in treating soft-tissue sarcoma. In another aspect, the invention relates to the use of AhR pathway in *in vitro* method of selecting for identifying new candidate substance or test compound for inhibiting neurofibroma growth and/or transformation to MPNST, or soft-tissue sarcoma treatment.

### INTRODUCTION

Cancers remain one of the most deadly threats to human health. Soft-tissue sarcomas are uncommon and represent about 1% of all new cancer cases each year. In 2010 about 3,272 new case of soft-tissue sarcoma were diagnosed in United Kingdom. The survival rate for 10 or more years is around 45%. More precisely, malignant peripheral nerve sheath tumors (MPNST) are aggressive soft-tissue sarcomas of neural origin and represent about 5%-10% of all soft-tissue sarcomas. In 50% of cases, they occur in the context of neurofibromatosis of type I, characterized by the loss of function, due to mutations, of tumor suppressor neurofibromin (NF1).

Neurofibromatosis type I is an autosomal dominant neurocutaneous genetic syndrome (Friedman et al. 1999). NF1 patients have an increased risk of both benign and malignant tumors; therefore, NF1 is classified as a tumor predisposition syndrome. The most common tumors are benign peripheral nerve sheath tumors, which may be dermal or plexiform type (Sabbagh et al. 2009). Dermal neurofibromas are typically small and grow as discrete lesions in the dermis whereas plexiform neurofibromas can develop internally along the plexus of major peripheral nerves and become quite large. Plexiform neurofibromas also called neurofibrosarcoma are probably derived from embryonic Schwann-cell lineage (Zhu et al. 2002). They can undergo malignant transformation to MPNST in about 10% of NF1 patients (Tucker et al. 2005).

Unfortunately, MPNST are resistant to conventional therapies, and their deep-seated position and locally invasive growth hinder complete surgical resection. Prognosis is generally poor because of low response rate to chemotherapy and propensity for rapid disease progression (Farid et al. 2014). Otherwise, the life expectancy of patients with dermal neurofibroma is slightly reduced compared to healthy population due to the increased risk of cancer. Currently, there is no specific treatment both for neurofibroma or MPNST. The only treatment concerns complications of the disease and relies essentially on surgical treatment.

There is still a need for new targets for the prevention and inhibition of neurofibroma growth, transformation to MPNST and for the treatment of soft-tissue sarcoma.

### SUMMARY OF THE INVENTION

The inventors have discovered the AhR pathway as a new target both for the prevention of neurofibroma growth and transformation to MPNST or for the treatment of soft-tissue sarcoma.

On that basis, the present invention provides a direct or indirect inhibitor of AhR pathway for use in preventing neurofibroma growth and/or transformation to MPNST.

According to a second aspect, the present invention provides a direct or indirect inhibitor of AhR pathway for use in treating soft-tissue sarcoma. In another embodiment, inventors propose an *in vitro* method of selecting candidate compound useful in preventing neurofibroma growth and/or transformation to MPNST, or treating soft-tissue sarcoma, said method comprising determining whether the substance or the compound is a direct or indirect inhibitor of AhR pathway.

The inhibitor of AhR pathway of the present invention is an inhibitor of AhR or AhR Nuclear Translocator (ARNT) activity or expression such as an antagonist of AhR, a shRNA or a siRNA.

Alternatively, said inhibitor is an indirect inhibitor which increases the activity or expression of AhRR (AhR Repressor) and/or inhibits the activity or expression of enzymes that produce AhR ligands, such as enzymes involved in the tryptophan metabolism which synthesize kynurenine, an endogenous ligand of AhR. In a particular aspect said enzymes are IDO 1 (Indoleamin 2,3-Dioxygenases 1), IDO 2 (Indoleamin 2,3-Dioxygenases 2), or TDO 2 (Tryptophan Dioxygenase 2).

According to another embodiment, the present invention relates to an *in vitro* method for determining the therapeutic efficiency of said inhibitor of AhR pathway in a treated soft-tissue sarcoma patient, comprising the comparison of a first biological sample of said soft-tissue sarcoma patient taken before the beginning of the treatment and a second corresponding biological sample of cancer patient after the beginning of the treatment. To determine the therapeutic efficiency of the treatment administered to the patient, the inventors compare the expression of genes of AhR pathway before and after the beginning of said treatment.

### BRIEF DESCRIPTION OF FIGURES

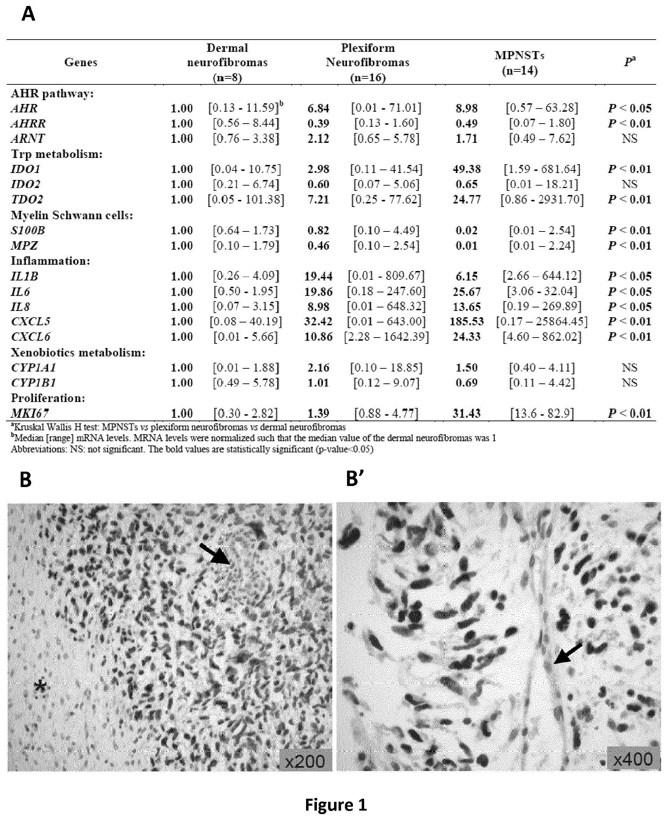

**Figure 1****: AHR signaling pathway is activated in Dermal and Plexiform Neurofibromas, and MPNST.**
   A, Total RNA was isolated from patients having Dermal Neurofibromas (n=8), Plexiform Neurofibromas (n=16) and MPNST (n=14). RT-qPCR were performed using primers recognizing specifically the genes indicated. Table indicates median [range] mRNA levels. mRNA levels were normalized such that the median value of the dermal neurofibromas was 1. Kruskal Wallis H test was used to compare MPNST vs Plexiform Neurofibromas vs Dermal Neurofibromas.
   B and B', Representative immunohistochemical study of AhR in a case of MPNST. Formalin fixed, paraffin embedded MPNST samples were stained with Hematoxylin-eosin-saffron (HES) and antibody recognizing AhR has been used to detect AhR. A strong nuclear AhR staining is detected in a proportion of tumor cells while intra-tumoral vessels (arrows) whereas fibroblasts in a fibrous septa (*) taken as internal negative controls are not stained.

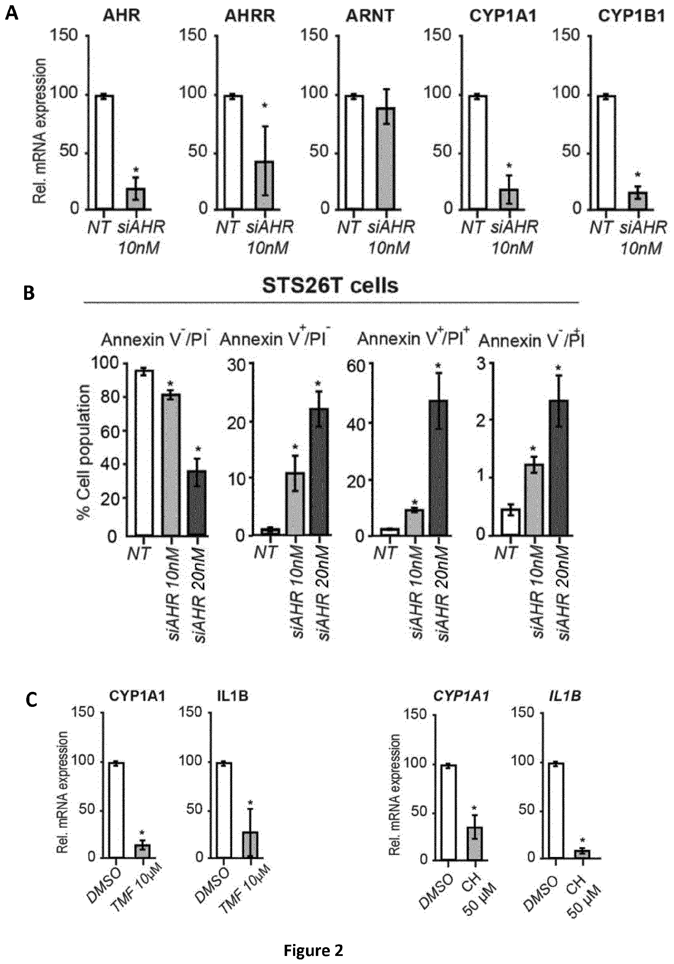

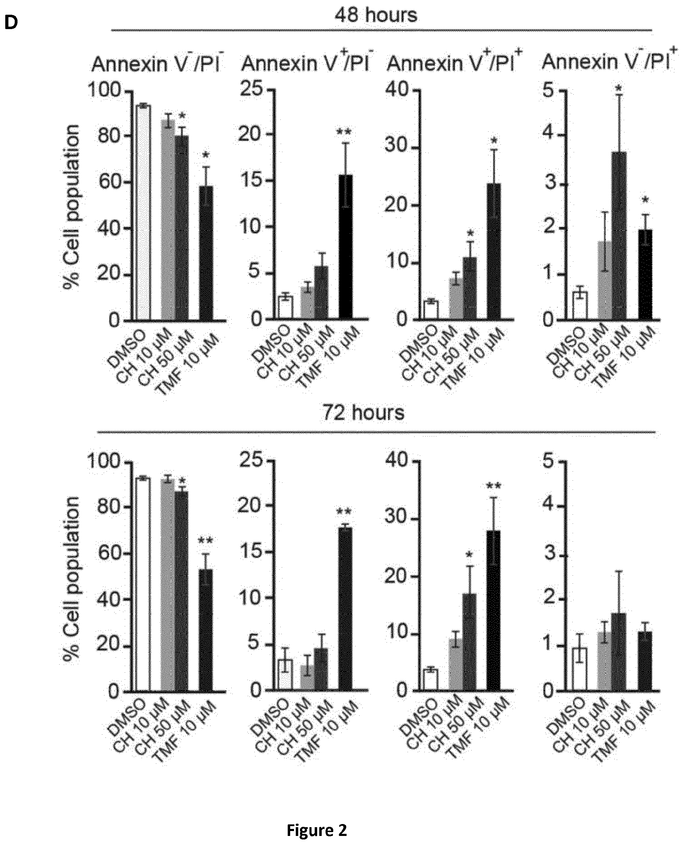

**Figure 2****: AhR inhibition induced death of MPNST cell lines (STS26T) by apoptosis.**
   A, STS26T cell line was reverse transfected with either non-transfected (NT) or siRNA (10 nM). The total RNA was then extracted from these cells. Quantitative real-time PCR experiments were performed using primers recognizing AhR, AHRR, ARNT, CYP1A1 and CYP1 B1. *p<0.05, by non-parametric Student's t-test.
   B, Flow cytometry has been conducted to determine the percentage of cell population of living cells, apoptotic cells, late apoptosis and dead. After 72h of medium change, cells are stained with Annexin and PI to detect the apoptosis and necrosis. Results represent the means +/- SEM of at least four independent experiments. *p<0.05, by non-parametric Student's t-test.
   C, Total RNA was extracted from MPNST cell line STS26T treated either with TMF (10µM) and CH-223191 (50µM) or DMSO. Quantitative real-time PCR experiments were performed using primers recognizing CYP1A1, IL1β. The RT-PCR was normalized using TBP mRNA. Data represent the mean +/- SEM of at least 3 independent experiments. *p<0.05, by Student's t-test.
   D, Human STS26T cell line was treated with either AHR antagonists CH-223191 (10µM and 50µM) or TMF (10µM) or DMSO. After 48h/72h of medium change, cells are stained with Annexin V and Propidium Iodide (PI) to detect apoptosis and necrosis. Flow cytometry has been conducted to determine the percentage of cell population of living cells (Annexin V-/PI-), apoptotic cells state (Annexin V+/PI-), late apoptosis state cells (Annexin V+/PI+) and dead cells (Annexin V-/PI+). Results represent the mean +/- SEM of at least four independent experiments. *p<0.05, **p<0.01, by Bonferroni's post hoc tests after one-way ANOVA when compared with DMSO.

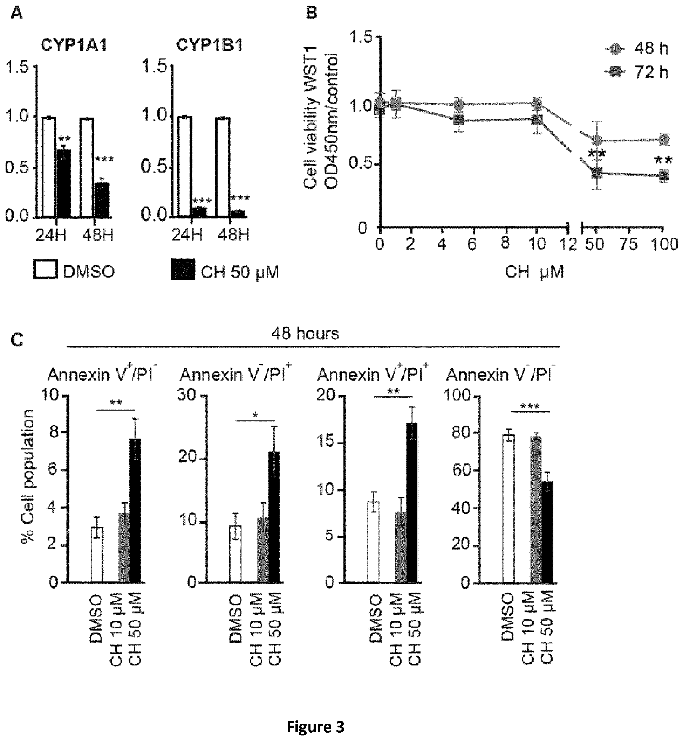

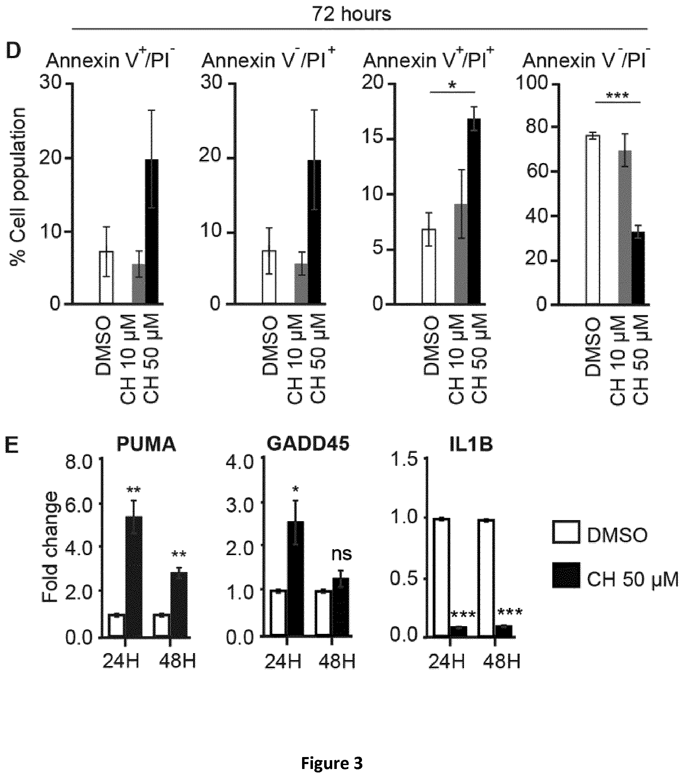

**Figure 3****: AhR inhibition induced death of MPNST cell lines (90-8) by apoptosis.**
   A, Total RNA was extracted from 90-8 cell line incubated with CH-223191 (50 µM). Quantitative real-time PCR experiments were performed using primers recognizing CYP1A1 and CYP1 B1. *p<0.05, **p<0.01, ***p<0.001 by students t-test.
   B, Human MPNST cell line (90-8) was incubated with AhR antagonist CH-223191 (1, 5, 10, 50 and 100 µM) during 48h and 72h. Cell viability was analyzed using WST1 assay. **p<.01, by Bonferroni's post hoc tests after two-way ANOVA when compared with DMSO.
   C - D, Percentage of cell population of apoptotic (Annexin V+/PI-), dead (Annexin V-/PI+), necrotic (Annexin V+/PI+) and living (Annexin V-/PI-) cells were analyzed using flow cytometry. Results represent the means +/- SEM of at least four independent experiments. *p<0.05, **p<0.01, ***p<0.001, by Bonferroni's *post hoc* tests after one-way ANOVA when compared with DMSO. E, Total RNA was extracted from 90-8 cell line. Quantitative real-time PCR experiments were performed using primers recognizing PUMA, GADD45 and IL1β. The RT-PCR was normalized using 26S RNA. Data represent the mean+/-SEM of at least 3 independent experiments. *p<0.05, **p<0.01, **p<0.001, by Student's t test.

### DESCRIPTION OF THE INVENTION

The invention relies in the surprisingly finding by the inventors of the implication of AhR pathway in neurofibromas growth and/or transformation to MPNST.

Accordingly, the invention relates to an inhibitor of aryl hydrocarbon receptor (AhR) pathway, said inhibitor directly inhibits AhR pathway or indirectly inhibits AhR pathway. The present invention is used in preventing neurofibroma growth and/or transformation to Malignant Peripheral Nerve Sheath Tumors (MPNST). The invention also relates to said direct or indirect inhibitor for use in treating soft-tissue sarcoma. The invention also relates to methods for identifying and selecting new candidate substance or compound for neurofibroma growth and/or transformation to MPNST, or soft-tissue sarcoma treatments using AhR pathway.

### Definitions

The terms "subject", "individual" and "patient" are herein used interchangeably and refer to a mammal suffering or suspected to suffer from soft-tissue sarcoma, more precisely a MPNST or likely to have neurofibroma.

Said mammals are preferably humans, but also include horses, cattles, sheeps, goats, or pets such as dogs and cats, or mammals used as laboratory models for human diseases, e.g. mouse, rat, rabbit, dog, cat, etc.

The term "biological sample" refers to any sample of the patient in which AhR expression can be measured, such as determining the level of RNA or determining the level of protein present in said biological sample. Such samples include notably a biopsy, a blood sample, a serum sample, a urine sample. For comparison purpose, the first and second samples should preferably be of the same nature (e.g. two tumor samples, two blood samples, two serum samples, or two urine samples).

The term "treatment" or "treating" refers to an action, application or therapy, wherein a subject, including a human being, is subjected to medical care with the purpose of improving the subject's condition, directly or indirectly. Particularly, this term refers to improving symptoms, preventing recurrence, improving prognosis, or even combination thereof, of soft-tissue sarcoma. The treatment may be curative or at least may result in alleviation of one or more of the symptoms of the disease.

The term "preventing" refers to an action or application, wherein a subject, including a human being, is subjected to medical care to prevent or relieve the occurrence of disease, particularly neurofibroma growth and/or transformation to MPNST.

### Inhibitor of AhR pathway

The Aryl Hydrocarbon Receptor (AhR) is a cytosolic ligand-activated transcription factor. It is considered as a mediator of the toxic effects of xenobiotics such as dioxins. Nevertheless, its endogenous functions only begin to emerge (Long et al. 2015) and natural ligands of the AhR, like tryptophan derivatives, have been identified. In fact, the AhR is present in ancient invertebrate organisms like nematodes, for which it is responsible for the regulation of dendritic arborization in the nervous system (Huang et al. 2004). Kynurenine is a tryptophan catabolite generated via Tryptophan Dioxygenase (TDO) and Indoleamin 2,3-Dioxygenases (IOD). By activating AhR pathway, kynurenine promotes tumor-cell survival and malignancy of glioma (Opitz et al. 2011). The activation of AhR provokes its heterodimerization with AhR Nuclear Translocator (ARNT). Then, the heterodimer binds specific responsive elements called Xenobiotic responsive elements (XREs), located in the promoter regions of the target genes and consequently, leading to their transactivation (i.e. Cytochrome 1A1 -CYP1A1...).

AhR begins to be an interesting area of research for scientist community. AhR has been found to be involved in functional interactions with signaling pathway governing cell proliferation, cell cycle, cell morphology, cell adhesion and cell migration. In fact, AhR modulates the balance between differentiation and pluripotency in normal and transformed tumor cells (Mulero-Navarro et al. 2016).

The Aryl-hydrocarbon Receptor Repressor (AhR Repressor) is a protein which participates in the aryl hydrocarbon receptor (AhR) signaling cascade involved in regulation of cell growth and differentiation. It functions as a feedback modulator by repressing AhR-dependent gene expression. It represses the transcription activity of AhR by competing with the heterodimer AhR/ARNT for the binding to the xenobiotic response element (XRE) sequence. XRE is present in the promoter regulatory region of variety of genes and thereby mediates the transcription activity of AhR.

The AhR Nuclear Translocator (ARNT) is a protein containing a basic helix-loop-helix domain and two characteristic PAS domains along with a PAC domain. The protein binds to ligand-bound aryl hydrocarbon receptor and aids in the movement of this complex to the nucleus, where it promotes the expression of genes involved in xenobiotic metabolism. This protein is also a co-factor for transcriptional regulation by hypoxia-inducible factor 1 (HIF-1).

In this context, WO 2012/015914 discloses novel agents that modulate AhR activity for inhibiting cancer cell proliferation and tumor cell invasion and metastasis, preferably in breast cancer.

In the present invention, the term "AhR inhibitor" means a substance, a compound or a molecule that decreases or inhibits the function associated to AhR pathway. This action can be effective directly or indirectly. The term "substance" refers to any chemically synthesized or naturally derived material or compound.

The terms "directly inhibits" means an action wherein a substance, a compound or a molecule may directly couple with AhR or ARNT to inhibit the physiological function of AhR pathway, more precisely said substance, compound or molecule is an inhibitor of AhR or ARNT activity or expression.

The terms "indirectly inhibits" means an action wherein the physiological function of AhR pathway is inhibited without direct couple with AhR or ARNT. For example, the substance, compound or molecule may increase the activity or expression of AhRR (AhR Repressor), or inhibits the activity or expression of IDO1 (Indoleamine 2,3-dioxygenase), IDO2 (Indoleamine 2,3-dioxygenase 2), or TDO2 (Tryptophan 2,3-dioxygenase) which inhibit AhR activity or expression.

The term "antagonist" means a receptor ligand or a drug that blocks or dampens a biological response by binding to a receptor. Antagonists mediate their effects by binding to the active orthosteric site or to allosteric sites on receptors, or they may interact at unique binding sites not normally involved in the biological regulation of the receptor's activity. Antagonist activity may be reversible or irreversible depending on the longevity of the antagonist-receptor complex, which, in turn, depends on the nature of antagonist-receptor binding. The majority of drug antagonists achieve their potency by competing with endogenous ligands or substrates at structurally defined binding sites on receptors.

Examples of the compound used as an AhR inhibitor include, without limitation, CH-223191, GNF351, StemRegenin 1, TMF, PDM2, α-Naphthoflavone.

Table 1 below lists examples of AhR inhibitor according to the invention.

**Table 1. Compound useful as AhR inhibitor.**

| **Generic Name** | **Molecule Name** | **Reference or CAS Number** |
|---|---|---|
| CH-223191 | 1-Methyl-N-[2-methyl-4-[2-(2-methylphenyl)diazenyl]phenyl-1H-pyrazole-5-carboxamide | 301326-22-7 |
| GNF351 | N-(2-(1H-Indol-3-yl)ethyl)-9-isopropyl-2-(5-methylpyridin-3-yl)-9H-purin-6-amine | Smith, K.J., et al. 2011. J. Pharmacol. Exp. Ther. 338, 318. |
| Stem Regenin 1 | 4-[2-[[2-(1-benzothiophen-3-yl)-9-propan-2-ylpurin-6-yl]aminolethyl]phenol | 1227633-49-9 |
| TMF | 6,2',4',-trimethoxyflavone | 720675-90-1 |
| PDM2 | 1,3-dichloro-5-[(1E)-2-(4-chlorophenyl)ethenyl]-benzene | 688348-25-6 |
| α-Naphthoflavone | 7,8-Benzoflavone | 604-59-1 |

In a specific aspect, the inhibitor is selected from the group consisting of CH-223191 or TMF.

Such inhibitors include antisense nucleic acid that inhibits the expression of AhR, such nucleic acids are well known to those persons skilled in the art and knows how to build them. Said nucleic acid include, preferably, antisense DNA, to the AhR gene, an interferent RNA (iRNA) or a ribozyme (Wu et al., 2008). In a particular embodiment, the AhR production or expression inhibitor is selected from the group consisting of antisense DNA to the AhR gene and an interferent RNA (iRNA). The term "iRNA" means any RNA which is capable of down-regulating the expression of the targeted gene. It encompasses small interfering RNA (siRNA), double-stranded RNA (dsRNA), single-stranded RNA (ssRNA), micro-RNA (miRNA), and short hairpin RNA (shRNA) molecules. RNAi can comprise naturally occurring RNA, synthetic RNA, or recombinantly produced RNA, as well as altered RNA that differs from naturally-occurring RNA by the addition, deletion, substitution and/or alteration of one or more nucleotides. Such alterations can include addition of non-nucleotide material, such as to the end of the molecule or to one or more internal nucleotides of the RNAi, including modifications that make the RNAi resistant to nuclease digestion. RNAi may be administered in free (naked) form or by the use of delivery systems that enhance stability and/or targeting, e.g., liposomes, or incorporated into other vehicles, such as hydrogels, cyclodextrins, biodegradable nanocapsules, bioadhesive microspheres, or proteinaceous vectors (WO 00/53722), or in combination with a cationic peptide (US 2007275923). They may also be administered in the form of their precursors or encoding DNAs. Preferably, the RNAi molecule is a siRNA of at least about 15-50 nucleotides in length, more preferably about 20-25 nucleotides in length. siRNA are usually designed against a region of 50-100 nucleotides downstream the translation initiator codon, whereas 5' UTR (untranslated region) and 3' UTR are usually avoided. The chosen siRNA target sequence should be subjected to a BLAST search against EST database to ensure that only the desired gene is targeted. An example of siRNA useful as an inhibitor of AhR is 5'-GCAAGUUAAUGGCAUGUUU-3' (SEQ ID NO:1), 5'-GAACUCAAGCUGUAUGGUA-3' (SEQ ID NO:2), 5'-GCACGAGAGGCUCAGGUUA-3' (SEQ ID NO:3), 5'-GCAACAAGAUGAGUCUAUU-3' (SEQ ID NO:4). Antisense nucleic acid can be complementary to all or part of a sense nucleic acid encoding the target gene product e.g., complementary to the coding strand of a double-stranded cDNA molecule or complementary to an mRNA sequence, and it thought to interfere with the translation of the target mRNA. An antisense nucleic acid can be, for example, about 5, 10, 15, 20, 25, 30, 35, 40, 45 or 50 nucleotides in length. Particularly, antisense RNA molecules are usually 18-50 nucleotides in length. An antisense nucleic acid for use in the methods of the invention can be constructed using chemical synthesis and enzymatic ligation reactions using procedures known in the art. Antisense nucleic acid may be modified to have enhanced stability, nuclease resistance, target specificity and improved pharmacological properties. Ribozymes are catalytic RNA molecules with ribonuclease activity, which are capable of cleaving a single-stranded nucleic acid, such as an mRNA, to which they have a complementary region. Thus, ribozymes can be used to catalytically cleave mRNA transcripts to thereby inhibit translation of the protein encoded by the mRNA. Ribozyme molecules specific for a gene product can be designed, produced, and administered by methods commonly known to the art (see e.g., Fanning and Symonds, 2006, RNA Towards Medicine (Handbook of Experimental Pharmacology), ed. Springer p. 5 289-303, reviewing therapeutic use of hammerhead ribozymes and small hairpin RNA). Alternately, AhR inhibitor include Cas9 guided RNA or an aptamer. Cas9 (CRISPR associated protein 9) is an RNA-guided DNA endonuclease, that is, an enzyme specialized for cutting DNA with two active cutting zones, one for each strand of the double helix. The Cas9 protein is associated with the adaptive immune system type II of CRISPR (Clustered Regular Interspaced Short Palindromic Repeats). This enzyme can be used in genetic engineering to easily and quickly modify the genome of animal and plant cells. Tools to edit the genome existed since the 1970s but were much less efficient, more complex and much more expensive than Cas9.

The terms "Aptamer" refers to a modified oligonucleotide selected from a random bank of sequences according to its affinity for a biological ligand intended for its therapeutic use. Aptamers differentiate from other nucleic acid sequences by their capacity to fold into a tertiary structure to create a binding pocket making it possible to interact precisely and specifically with the target. Aptamers have numerous therapeutic applications (Kaur and Roy, 2008)

In a particular aspect of the invention, the inhibitor is selected from the group consisting of 5'-GCAAGUUAAUGGCAUGUUU-3' (SEQ ID NO:1), 5'-GAACUCAAGCUGUAUGGUA-3' (SEQ ID NO:2), 5'-GCACGAGAGGCUCAGGUUA-3' (SEQ ID NO:3), 5'-GCAACAAGAUGAGUCUAUU-3' (SEQ ID NO:4).

Alternately, the inhibitor is a modulator of the kynurenine synthesis. Indeed, kynurenine is an endogenous ligand of AhR Therefore, kynurenine is a good target for the inhibition of AhR pathway.

In another aspect, the inhibitor increases the activity or expression of AhRR (AhR Repressor), or inhibits the activity or expression of enzymes that produce AhR ligands, such as enzymes involved in the tryptophan metabolism which synthesize kynurenine, an endogenous ligand of AhR. In a particular aspect said enzymes are IDO 1 (Indoleamin 2,3-Dioxygenases 1), IDO 2 (Indoleamin 2,3-Dioxygenases 2), or TDO 2 (Tryptophan Dioxygenase 2).

The Indoleamine 2,3-dioxygenase (IDO 1) is a heme enzyme that catalyzes the first and rate-limiting step in tryptophan catabolism to N-formyl-kynurenine. This enzyme acts on multiple tryptophan substrates including D-tryptophan, L-tryptophan, 5-hydroxy-tryptophan, tryptamine, and serotonin. This enzyme is thought to play a role in a variety of pathophysiological processes such as antimicrobial and antitumor defense, neuropathology, immunoregulation, and antioxidant activity. Through its expression in dendritic cells, monocytes, and macrophages this enzyme modulates T-cell behavior by its peri-cellular catabolization of the essential amino acid tryptophan. An important paralog of IDO1 is Indoleamine 2,3-dioxygenase 2 (IDO 2).

The Tryptophan 2,3-Dioxygenase (TDO2) is a heme enzyme that plays a critical role in tryptophan metabolism by catalyzing the first and rate-limiting step of the kynurenine pathway. Increased activity of the encoded protein and subsequent kynurenine production may also play a role in cancer through the suppression of antitumor immune responses, and single nucleotide polymorphisms in this gene may be associated with autism.

As exemplified in the experimental section, from the surprising discovery that AhR pathway is specifically altered in human Schwann cell tumors, inventors have been able to identify specific targets and their modulators to be used for treating or preventing neurofibroma, soft-tissue sarcoma or MPNST. More particularly, inventors show that silencing of AhR gene as well as small compounds known as antagonists of AhR provoke death of several MPNST cell lines.

### Therapeutic uses

Accordingly, the invention related to an inhibitor as defined above for use in preventing neurofibroma growth and/or transformation to Malignant Peripheral Nerve Sheath Tumors (MPNST). In another aspect, the invention related to an inhibitor for use in treating soft-tissue sarcoma.

More specifically, an object of this invention related in inhibitor of AhR, ARNT, AhRR.

The inhibitor of AhR pathway is an antagonist of AhR, said antagonist is selected from the group consisting of CH-223191, GNF351, StemRegenin 1, TMF, PDM2, α-Naphthoflavone, more preferably, CH-223191 or TMF.

The inhibitor of AhR pathway may also be a siRNA, preferably, a siRNA selected from the group consisting of SEQ ID NO:1, SEQ ID NO:2, SEQ ID NO:3, SEQ ID NO:4.

Even more specifically, an object of this invention related in modulator of tryptophan metabolism, which is involved in synthesis of kynurenine, an endogenous ligand of AhR. In a particular aspect said enzymes are IDO 1, IDO 2, or TDO 2.

According to another aspect, the present invention relates to a method for treating or preventing a disorder in a subject in need thereof, comprising administering to said subject a substance or a compound of the present invention, more precisely an inhibitor of AhR pathway.

In an embodiment, the AhR pathway inhibitors are intended for preventing neurofibroma growth and/or transformation to Malignant Peripheral Nerve Sheath Tumors (MPNST). Indeed, Malignant Peripheral Nerve Sheath Tumors are strongly associated with internal plexiform neurofibromas. Thus, patients with neurofibromatosis type 1 and more particularly subcutaneous neurofibromas were approximately 3 times more likely to have internal plexiform neurofibromas or MPNSTs than individuals without internal plexiform neurofibromas. In addition, patients with internal plexiform neurofibromas were 20 times more likely to have MPNSTs than individuals without internal plexiform neurofibromas (Tucker et al. 2005).

In another particular embodiment, the present invention relates to an *in vitro* method for determining the therapeutic efficiency of the above treatments with at least an inhibitor of AhR pathway in a treated subject in need thereof, from a first biological sample of said cancer patient taken before the beginning of the treatment and a second corresponding biological sample of cancer patient after the beginning of the treatment, comprising:
a) Measuring AhR expression level in said first and second biological samples;
b) Comparing at least the (activated) AhR expression levels; and
c) Determining the efficiency of the treatment with an inhibitor of AhR pathway in said treated patient with soft-tissue sarcoma from said comparison, the treatment being considered efficient if AhR expression level measured in the biological sample after the beginning of the treatment is lower than AhR expression level in the biological sample before the beginning of the treatment.

The AhR inhibitors are useful in treating or preventing a cancer related to an activation of AhR, more particularly neurofibroma, or soft-tissue sarcoma or MPNST.

According to the invention, the AhR pathway inhibitors have a beneficial impact by blocking AhR signaling in MPNST cells in order to dampen their progression. Interestingly, antagonist of AhR, provoked the death of two MPNST cell lines: 90-8 and STS26T.

According to the invention, inventors further analyzed other genes linked to the AhR signaling pathway and involved in tryptophan metabolism that lead to the synthesis of kynurenine, an endogenous ligand of AhR (Opitz et al. 2011; Mezrich et al. 2010; Zhai et al. 2015). Two genes participating to the first steps of tryptophan metabolism, namely IDO1 and TDO2, were significantly increased in plexiform neurofibromas and MPNST. The enzyme IDO1 mediates the first, rate-limiting, step in tryptophan metabolites to kynurenine, and is up-regulated under an inflammatory microenvironment (Litzenburger et al. 2014). Through generation of downstream metabolites, IDO1 enzyme activity may also affect immunity, including specific immunomodulatory or cytotoxic functions (Mezrich et al. 2010). Inventors showing a progressive up-regulation of IDO1 and TDO2 in plexiform neurofibromas (3-fold and 5-fold), and a further stimulation in MPNST (50-fold and 25-fold) indicates that tryptophan metabolism may play a role in peripheral nerve sheath tumor progression to malignancy.

### Screening methods

In another aspect, the implication of AhR pathway is of a particular use in the search for candidate compounds for treating or preventing neurofibroma, soft-tissue sarcoma or MPNST. Indeed, as exemplified in the experimental section inhibition of this pathway lead to the death of tumors cells. Hence, AhR expression level is valuable marker for selecting candidate compounds to be further studied for their use in the treatment of soft-tissue sarcoma.

Accordingly, the present invention provides an *in vitro* method of selecting candidate compound useful in preventing neurofibroma growth and/or transformation to MPNST, or treating soft-tissue sarcoma, said method comprising determining whether a substance or a compound is a direct or indirect inhibitor of AhR pathway. The method may be an *in vivo, ex vivo* or *in vitro* method, preferably an *in vitro* method.

In an embodiment, said method comprises the steps of:
(i) measuring the expression or the activity of AhR, in the presence and absence of said candidate substance or said compound and
(ii) selecting said candidate substance or compound having the ability to inhibit directly or indirectly the expression or activity of AhR pathway, as a candidate useful in preventing neurofibroma growth and/or transformation to MPNST, or treating soft-tissue sarcoma.

In a preferred embodiment, said substance or said compound, object of said method of selecting candidate compound is an inhibitor of AhR, ARNT, IDO1, IDO2, TDO2 or an activator of AhRR.

In further step, the candidate inhibitors can be subjected to *in vitro* testing on cell lines of neurofibroma, or MPNST or soft-tissue sarcoma.

The Examples and Figures illustrate below the invention without limiting its scope.

### Examples

### Materials and methods

**Table 2: Primers used for the experiments.**

| **Primers** | **Sequences** |
|---|---|
| AhR | F : 5'GCCTTGGTCTTCTATGCTCT3' |
| | R: 5'GCTTTGTGCTGGTTGTCAC3' |
| 26S | F 5'-AGGAGAAACAACGGTCGTGCCAAAA - 3' |
| | R 5'-GCGCAAGCAGGTCTGAATCGTG - 3' |

| **Primers for clinical samples** | |
|---|---|
| AHR-U | F 5'-TAA CCC AGA CCA GAT TCC TCC AGA-3' |
| AHR-L | R 5'-CCC TTG GAA ATT CAT TGC CAG A -3' |
| AHRR | F 5'-GGA AGG CTG CTG TTG GAG TCT CT-3' |
| AHRR-L | R5'- TGG AAG CCC AGA TAG TCC ACG A-3' |
| TBP-U | F5'-TGC ACA GGA GCC AAG AGT GAA-3' |
| TBP-L | R 5'-CAC ATC ACA GCT CCC CAC CA-3' |

### Cell culture

The MPNST-derived cell line STS26T was maintained in advanced RPMI-1640 with 15% heat-inactivated FBS, 2.75‰ Bovine Pituitary Extract, 100U/ml penicillin, and 100µg/ml streptomycin (Invitrogen). MPNST cell line 90-8 was established from NF1-associated MPNST: 90-8 was kindly provided by Pr. N. Ratner (Cincinnati Children's Hospital Medical). MPNST cell line STS26T was established from a sporadic MPNST and was kindly provided by G. H. De Vries (Hines VA Hospital, Illinois, USA) (Nagarajan R et al. 2005).

### Plasmids and chemicals

The AhR was targeted by four siRNAs recognizing four different regions of AhR transcript. An example of siRNA useful as an inhibitor of AhR is 5'-GCAAGUUAAUGGCAUGUUU-3' (SEQ ID NO:1), 5'-GAACUCAAGCUGUAUGGUA-3' (SEQ ID NO:2), 5'-GCACGAGAGGCUCAGGUUA-3' (SEQ ID NO:3), 5'-GCAACAAGAUGAGUCUAUU-3' (SEQ ID NO:4). The siRNA that inventors used did not have any off-target effect. TCDD was purchased from LGC standards and CH-223191, TMF from Sigma.

### Quantitative RT-PCR experiments

Total RNA from cultured MSC80 was obtained using TRIzol® Reagent (Invitrogen, France): 1 µg of total RNA was reverse transcribed with random primers from Promega (Charbonnières, France) and reverse transcriptase M-MLV-RT from Invitrogen (Cergy Pontoise, France). Quantitative real time PCR was performed with standard protocols using SYBR®Green ROX Mix (Thermo scientific, France) as a fluorescent detection dye in ABI PRISM® 7000 in a final volume of 10 µl which also contains 300 nM primers (Operon, Cologne, Germany) and 20 ng of reverse transcribed RNA in 384-well plates. To characterize the generated amplicons and to control the contamination by unspecific byproducts, a melting curve analysis was employed. Each reaction was performed in triplicate and the mean of at least three independent experiments was calculated. All results were normalized to the 26S mRNA level and calculated using the Delta Ct method. The primer sequences used in real time qPCR are listed in the primers table (Table 2).

### Patients and Samples

Samples were obtained by laser excision (dermal neurofibromas) or surgical excision (plexiform neurofibromas and MPNST) from patients with neurofibromatosis type 1. Each patient signed a written informed consent form, and this study was approved by the local ethic committee.

### Immunohistochemical study on human samples

Formalin fixed, paraffin embedded (FFPE) MPNST samples were retrieved from the archive material of the department of Pathology of Cochin hospital. Hematoxylin-eosin-saffron (HES) staining and immunohistochemistry were applied to 3 micrometer-thick dewaxed sections. Immunostaining of AHR (rabbit polyclonal antibody clone H211, Santa Cruz) was done at 3 µg/ml after heat antigen retrieval in pH 6.0 citrate buffer. In all samples, inventors checked the absence of staining in intra-tumoral vessels and fibroblasts as internal negative controls.

### Expression of genes involved in the AhR signaling pathway in patients' samples

Inventors used real-time quantitative RT-PCR (qRT-PCR) to quantify the mRNA expression level of 16 AhR signaling pathway associated genes in a series of 16 human plexiform neurofibromas, and 14 MPNST in comparison with 8 dermal neurofibromas, as well in a mouse cell line (MSC80). The real-time quantitative RT-PCR using the ABI Prism 7900 Sequence Detection System (Perkin-Elmer Applied Biosystems) have been described (Latil et al. 2001). Each sample was normalized on the basis of its TBP content. Results, expressed as N-fold differences in target gene expression relative to the TBP gene, and termed "Ntarget". Ntarget = 2ΔCtsample, where the ΔCt value of the sample was determined by subtracting the average Ct value of the target gene from the average Ct value of the TBP gene. The Ntarget values of the tumor samples were subsequently normalized such that the mean of the dermal neurofibroma Ntarget values was 1. The nucleotide sequences of the human primers used are listed in the primers table (Table 2). To avoid amplification of contaminating genomic DNA, one of the two primers was placed at the junction between two exons. Agarose gel electrophoresis was used to verify the specificity of PCR amplicons.

### In vitro cytotoxicity assay (WST1 assay) and cell counting

90-8 cells were seeded in triplicate on 96-well plates. Cells were treated with either CH-223191 or 0.1% DMSO. Cytotoxicity was quantified 48 and 72 hours after treatment using WST1 assay (Roche). Relative cytotoxicity was determined as ratio of absorbance at 450 nm of cells treated with CH-223191 versus cells treated with DMSO. IC50 values corresponding to concentrations associated to 50% of cell viability after 72 hours of continuous exposure were determined.

### Flow cytometry analysis

STS26T cells were cultured in 12-well plates and were treated with 10 µM, 50 µM of CH-223191, 10 µM of TMF, or 0.1 % DMSO and cisplatin (33 µM). Cells undergoing apoptosis at the indicated time were identified using Annexin V-FITC apoptosis detection kit (Abcam ab14085), following the manufacturer's instruction. Inventors used a BD FACSCanto™ II. Propidium iodide (PI) is a fluorescent intercalating agent and is used as a DNA stain to evaluate cell viability or DNA content in cell cycle analysis. At least 10,000 events were recorded at a light scatter to exclude debris and aggregate with a flow rate of less than 200 cells/second for each assay. Green fluorescence (FITC) and Yellow fluorescence (PE-A) were detected in green and yellow channel respectively. The compensations and the settings were adjusted according to the assay. Data were analyzed using FlowJo Software.

### Statistical analysis

Unless otherwise specified, means of treatment groups were compared with one-way analysis of variance (ANOVA). When the ANOVA showed that there were significant differences between the groups, Tukey's test was used to identify the sources of these differences. A P value of ≤ 0.05 was considered statistically significant. Two group comparisons were performed by the Student's t test.

For clinical samples, as the mRNA levels did not fit a Gaussian distribution, (a) the mRNA levels in each subgroup of samples were expressed as their median values and ranges, rather than their mean values and coefficients of variation, and (b) comparison between the three types of nerve sheath tumors were assessed by using the non-parametric Kruskal Wallis H test. Differences were considered significant at confidence levels greater than 95% (P < 0.05).

### Results

### AhR signaling pathway is activated in peripheral nerve sheath tumorigenesis

In a series of 38 tumors, inventors first analyzed mRNA expression of AhR, AhRR (AhR Repressor), and ARNT (Fig 1A). AhR was significantly upregulated in plexiform neurofibromas (7-fold) and in MPNST (9-fold), compared to the dermal neurofibromas. AhRR was significantly down-regulated in plexiform neurofibromas (3-fold) and MPNST (2-fold) compared to the dermal neurofibromas, while ARNT expression was not significantly altered in either the plexiform neurofibromas or the MPNST.

Several genes implicated in AhR pathway were also tested, including kynurenine metabolism (IDO1, IDO2, and TDO2), inflammation and immunity (IL1ß, IL6, IL8, CXCL5, and CXCL6) pathways, Schwann Cell markers (S100B, and P0) and proliferation (MKI67). IDO1, and TDO2 (but not IDO2) were significantly up-regulated in plexiform neurofibromas (3-fold to 7-fold) and in MPNST (50-fold and 25-fold, respectively). Concerning the inflammatory pathway: IL1β, IL6, IL8, CXCL5, and CXCL6 mRNA levels showed significant overexpression in plexiform neurofibromas and in MPNST, compared to the dermal neurofibromas (Fig. 1A). The proliferation-associated gene MKI67 was significantly overexpressed (31-fold) in the MPNST. It is noteworthy that, SC-specific genes (S100B, P0) expression was shown to be significantly down-regulated in MPNST suggesting a dedifferentiation of Schwann cells.

Inventors also performed an immunostaining of AhR in MPNST (Fig. 1B, B'). Inventors detected strong nuclear AhR staining in a proportion of tumor cells, while intra-tumoral vessels and fibroblasts in a fibrous septum (taken as internal negative controls) were not stained. Altogether, data show that AhR pathway is altered in human Schwann cell tumors.

### AhR inhibition induced death of MPNST cell lines.

Inventors established that AhR expression is increased in human plexiform neurofibromas and MPNST. Inventors hypothesized that targeting AhR could affect MPNST cells survival. Inventors knocked-down AhR in human MPNST derived cell line (STS26T). The silencing of AhR (10nM) drastically decreased the expression of AhR and its target genes cyp1A1 and cyp1B1 as well as AhRR, without affecting the expression of ARNT (Fig. 2A). After the transfection with a siRNA against AhR (20 nM) and at 72h, STS26T cell viability was decreased by 60% as suggested by Annexin V and PI staining analyzed by flow cytometry (Fig. 2B). The number of apoptotic and necrotic cells represented 20% and 45% of total cells, respectively.

Inventors used two AhR antagonists (CH-223191 and TMF) to inhibit AhR activity in STS26T cells. As expected, TMF and CH-223191 inhibited the expression of AhR target gene: CYP1A(Fig. 2C). Treatment with CH-223191 (50µM at 48h and 72h) increased cell death and enhanced apoptosis and necrosis as quantified by Annexin V and PI staining (Fig. 2D). TMF (10µM at 48h and 72h) exhibited a more potent effect than CH-223191 on STS26T cell death. After 72h of TMF (10µM), inventors observed a 50% decrease of cell viability. 20% of the cells underwent apoptosis and 30% were necrotic.

These results were confirmed using another MPNST cell line 90-8. As expected, CH-223191 inhibited the expression of the classical AhR target genes: CYP1A1 and CYP1B1 (Fig. 3A). The incubation of 90-8 cells with CH-223191 (50 µM) decreased 90-8 cell viability by 50% as suggested by WST1 assay (Fig. 3B). Inventors dissected the mechanism by which AhR antagonist CH-223191 elicits 90-8 cell death. CH-223191 (50µM, 48h (Fig.3C) and 72h (Fig.3D)) treatment increased the number of dead cells and enhanced apoptosis and necrosis as quantified by Annexin V and PI staining. Finally, inventors also showed that CH-223191 enhanced the expression of PUMA and GADD45 genes involved in cell death and DNA damage (Fig. 3E). It also reduced the expression of IL1β that was elevated in MPNST tumors (Fig. 1A).

### Conclusion

The present data show that inhibition of AhR signaling, provokes the death of MPNST cells and indicate that AhR represents an interesting target for peripheral nerve sheath tumor therapy and for neurofibroma and peripheral nerve sheath tumor prevention.

### Reference

Friedman JM. Epidemiology of neurofibromatosis type 1. Am J Med Genet. 1999;89:1-6.
Sabbagh A, Pasmant E, Laurendeau I, Parfait B, Barbarot S, Guillot B, et al. Unravelling the genetic basis of variable clinical expression in neurofibromatosis 1. Hum Mol Genet. 2009;18:2768-78.
Zhu Y, Ghosh P, Charnay P, Burns DK, Parada LF. Neurofibromas in NF1: Schwann cell origin and role of tumor environment. Science. 2002;296:920-2.
Tucker T, Wolkenstein P, Revuz J, Zeller J, Friedman JM. Association between benign and malignant peripheral nerve sheath tumors in NF1. Neurology. 2005;65:205-11.
Farid M, Demicco EG, Garcia R, Ahn L, Merola PR, Cioffi A, Maki RG. Malignant peripheral nerve sheath tumors. Oncologist. 2014 Feb;19(2):193-201. doi: 10.1634/theoncologist.2013-0328. Epub 2014 Jan 27.
Long X, Li S, Xie J, Li W, Zang N, Ren L, et al. MMP-12-mediated by SARM-TRIF signaling pathway contributes to IFN-γ-independent airway inflammation and AHR post RSV infection in nude mice. Respir Res. 2015;16:11.
Huang X, Powell-Coffman JA, Jin Y. The AHR-1 aryl hydrocarbon receptor and its co-factor the AHA-1 aryl hydrocarbon receptor nuclear translocator specify GABAergic neuron cell fate in C. elegans. Development. 2004;131:819-28.
Opitz CA, Litzenburger UM, Sahm F, Ott M, Tritschler I, Trump S, et al. of the human aryl hydrocarbon receptor. Nature. 2011;478:1-7.
Mezrich JD, Fechner JH, Zhang X, Johnson BP, Burlingham WJ, Bradfield CA. An interaction between kynurenine and the aryl hydrocarbon receptor can generate regulatory T cells. J Immunol. American Association of Immunologists; 2010;185:3190-8.
Zhai L, Lauing KL, Chang AL, Dey M, Qian J, Cheng Y, et al. The role of IDO in brain tumor immunotherapy. J Neurooncol. 2015;123:395-403.
Litzenburger UM, Opitz CA, Sahm F, Rauschenbach KJ, Trump S, Winter M, et al. Constitutive IDO expression in human cancer is sustained by an autocrine signaling loop involving IL-6, STAT3 and the AHR. Oncotarget. 2014;5:1038-51.
Mulero-Navarro, Fernandez-Salguero. New Trends in Aryl Hydrocarbon Receptor Biology. Front Cell Dev Biol. 2016 May 11 ;4:45.
Le N, Nagarajan R, Wang JYT, Araki T, Schmidt RE, Milbrandt J. Analysis of congenital hypomyelinating Egr2Lo/Lo nerves identifies Sox2 as an inhibitor of Schwann cell differentiation and myelination. Proc Natl Acad Sci USA. 2005;102:2596-601.
Makoukji J, Shackleford G, Meffre D, Grenier J, Liere P, Lobaccaro J-MA, et al. Interplay between LXR and Wnt/β-catenin signaling in the negative regulation of peripheral myelin genes by oxysterols. J Neurosci. 2011;31:9620-9.
Massaad C, Garlatti M, Wilson EM, Cadepond F, Barouki R. A natural sequence consisting of overlapping glucocorticoid-responsive elements mediates glucocorticoid, but not androgen, regulation of gene expression. Biochem J. 2000 Aug 15;350 Pt 1:123-9.
Latil A, Bieche I, Vidaud D, Lidereau R, Berthon P, Cussenot O, et al. Evaluation of Androgen, Estrogen (ER{{alpha}} and ER{beta}), and Progesterone Receptor Expression in Human Prostate Cancer by Real-Time Quantitative Reverse Transcription-Polymerase Chain Reaction Assays. Cancer Res. 2001;61:1919-26.
Fanning and Symonds, 2006, RNA Towards Medicine (Handbook of Experimental Pharmacology), ed. Springer p. 5 289-303, reviewing therapeutic use of hammerhead ribozymes and small hairpin RNA
Kaur et al., "Therapeutic applications of aptamers," Expert Opin. Investig. Drugs, vol. 17, No. 1,pp.43-60,2008.
Wu et al., "Let me count the Ways: Mechanisms of Gene Regulation by miRNAs and siRNAs," Molecular Cell, vol. 29, pp. 1-7, Jan. 18, 2008.

## Claims

1. An inhibitor of aryl hydrocarbon receptor (AhR) pathway for use in preventing neurofibroma growth and/or transformation to Malignant Peripheral Nerve Sheath Tumors (MPNST).

2. An inhibitor of aryl hydrocarbon receptor (AhR) pathway for use in treating soft-tissue sarcoma.

3. The inhibitor for use according to claim 1 or 2, wherein said inhibitor is an inhibitor of AhR or ARNT (AhR Nuclear Translocator) activity or expression.

4. The inhibitor for use according to claim 1 or 2, wherein said inhibitor increases the activity or expression of AhRR (AhR Repressor), or inhibits the activity or expression of IDO1 (Indoleamine 2,3-dioxygenase) or IDO2 (Indoleamine 2,3-dioxygenase 2) or TDO2 (Tryptophan 2,3-dioxygenase).

5. The inhibitor for use according to claim 3, wherein said inhibitor of AhR is an antagonist of AhR.

6. The inhibitor for use according to claim 5, wherein said antagonist of AhR is selected from the group consisting of CH-223191, GNF351, StemRegenin 1, TMF, PDM2 and α-Naphthoflavone.

7. The inhibitor for use according to claim 6, wherein said inhibitor is CH-223191 or TMF.

8. The inhibitor for use according to claim 3, wherein said inhibitor of AhR is a siRNA.

9. The inhibitor for use according to claim 8 is selected from the group consisting of SEQ ID NO:1, SEQ ID NO:2, SEQ ID NO:3, SEQ ID NO:4.

10. An *in vitro* method of selecting candidate compound useful in preventing of neurofibroma growth and/or transformation to MPNST, or treating soft-tissue sarcoma, said method comprising determining whether a substance or a compound is a direct or indirect inhibitor of AhR pathway.

11. The method according to claim 10, which comprises the steps of:
(i) measuring the expression or the activity of AhR, in the presence and absence of said candidate substance or said compound and
(ii) selecting said candidate substance or compound having the ability to inhibit directly or indirectly the expression or activity of AhR pathway, as a candidate useful in preventing neurofibroma growth and/or transformation to MPNST, or treating soft-tissue sarcoma.

12. The method according to claim 11, wherein said substance or said compound is an inhibitor of AhR, ARNT, IDO1, IDO2, TDO2.

13. The method according to claim 11, wherein said substance or said compound is an activator of AhRR.

14. An *in vitro* method for determining the therapeutic efficiency of an inhibitor of AhR pathway in a treated patient with soft-tissue sarcoma, comprising the comparison of a first biological sample of said cancer patient taken before the beginning of the treatment and a second corresponding biological sample of cancer patient after the beginning of the treatment.


SEQUENCE LISTING

<110> UNIVERSITE PARIS DESCARTES (PARIS V)
      INSTITUT NATIONAL DE LA SANTE ET DE LA RECHERCHE MEDICALE
      (INSERM)
      ASSISTANCE PUBLIQUE-HOPITAUX DE PARIS (AP-HP)

<120> Use of inhibitors of aryl hydrocarbon receptor for treating
soft-tissue sarcoma and preventing neurofibroma growth and/or transformation
to Malignant Peripheral Nerve Sheath Tumors

<130> 407

<160> 14

<170> BiSSAP 1.3.6

<210> 1
<211> 19
<212> RNA
<213> Artificial Sequence

<220>
<223> AhR

<400> 1
gcaaguuaau ggcauguuu                                                    19

<210> 2
<211> 19
<212> RNA
<213> Artificial Sequence

<220>
<223> AhR

<400> 2
gaacucaagc uguauggua                                                    19

<210> 3
<211> 19
<212> RNA
<213> Artificial Sequence

<220>
<223> AhR

<400> 3
gcacgagagg cucagguua                                                    19

<210> 4
<211> 19
<212> RNA
<213> Artificial Sequence

<220>

<223> AhR

<400> 4
gcaacaagau gagucuauu                                                    19

<210> 5
<211> 20
<212> DNA
<213> Artificial Sequence

<220>
<223> AhR

<400> 5
gccttggtct tctatgctct                                                   20

<210> 6
<211> 19
<212> DNA
<213> Artificial Sequence

<220>
<223> AhR

<400> 6
gctttgtgct ggttgtcac                                                    19

<210> 7
<211> 25
<212> DNA
<213> Artificial Sequence

<220>
<223> 26S

<400> 7
aggagaaaca acggtcgtgc caaaa                                             25

<210> 8
<211> 22
<212> DNA
<213> Artificial Sequence

<220>
<223> 26S

<400> 8
gcgcaagcag gtctgaatcg tg                                                22

<210> 9
<211> 24
<212> DNA
<213> Artificial Sequence

<220>
<223> AHR-U

<400> 9
taacccagac cagattcctc caga                                              24


<210> 10
<211> 23
<212> DNA
<213> Artificial Sequence


<220>
<223> AHR-L

<400> 10
rcccttggaa attcattgcc aga                                               23


<210> 11
<211> 23
<212> DNA
<213> Artificial Sequence


<220>
<223> AHRR

<400> 11
ggaaggctgc tgttggagtc tct                                               23


<210> 12
<211> 23
<212> DNA
<213> Artificial Sequence


<220>
<223> AHRR-L

<400> 12
rtggaagccc agatagtcca cga                                               23


<210> 13
<211> 21
<212> DNA
<213> Artificial Sequence


<220>
<223> TBP-U

<400> 13
tgcacaggag ccaagagtga a                                                 21


<210> 14
<211> 20
<212> DNA

<213> Artificial Sequence

<220>
<223> TBP-L

<400> 14
cacatcacag ctccccacca                                                    20